# EUROPEAN PATENT APPLICATION

(11) **EP 4 674 367 A2**
(43) Date of publication of application: **07.01.2026**
(21) Application number: 23932181.3
(22) Date of filing: 11.04.2023
(51) Int. Cl.: A61B 18/12, A61B 18/00

(54) **RF ENERGY GENERATOR, AND METHOD AND COMPUTER PROGRAM FOR OUTPUTTING RF ENERGY TO ELECTRODE APPARATUS**

(30) Priority: 04.04.2023 KR 20230043869
(71) Applicant: Deepqure Inc., Seoul 06243 (KR)
(72) Inventor: HONG, Suk Ki, Seoul 06243 (KR); YOON, Young Il, Seoul 06243 (KR); BACH, Du Jin, Seoul 06243 (KR); KIM, Kee Wan, Seoul 06243 (KR); PARK, Chan Ho, Seoul 06243 (KR); PARK, Jong Sun, Seoul 06243 (KR)
(74) Representative: Manitz Finsterwald Patent- und Rechtsanwaltspartnerschaft mbB
(86) International application number: PCT/KR2023/004836
(87) International publication number: WO 2024/210246

(57) **Abstract**

An RF energy generator that outputs RF energy to an electrode apparatus for blocking or controlling nerves in body includes: a receiving unit configured to receive temperature data of a tube in a body from the electrode apparatus; a controller configured to calculate a temperature value of the tube in the body based on the received temperature data, calculate a temperature variance of the tube in the body based on the calculated temperature value of the tube in the body, and determine an intensity of RF energy through PID control based on the temperature variance of the tube; and an output unit configured to output the RF energy to the electrode apparatus based on the determined intensity of RF energy.

## Description

### TECHNICAL FIELD

The present disclosure relates to an RF energy generator that outputs RF energy to an electrode apparatus for blocking or controlling nerves in body, a method of outputting RF energy, and a computer program.

### BACKGROUND

Denervation is a surgical procedure intended to control an abnormally overactive autonomic nervous system by damaging specific nerves. For example, renal denervation can treat hypertension and heart diseases by damaging renal sympathetic nerves directed to the kidney, and pulmonary denervation can treat lung diseases by damaging parasympathetic nerves directed to the lung.

When the surgical procedure is performed as described above, it is important to accurately transmit electrical impulses required for the surgical procedure to nerves which are a procedure target. Specifically, in order to effectively denervate or modulate the nerves on a tube in a body, it is important to accurately transfer RF energy required for the surgical procedure depending on a state of the tube with distributed nerves (e.g., a temperature of the tube, etc.).

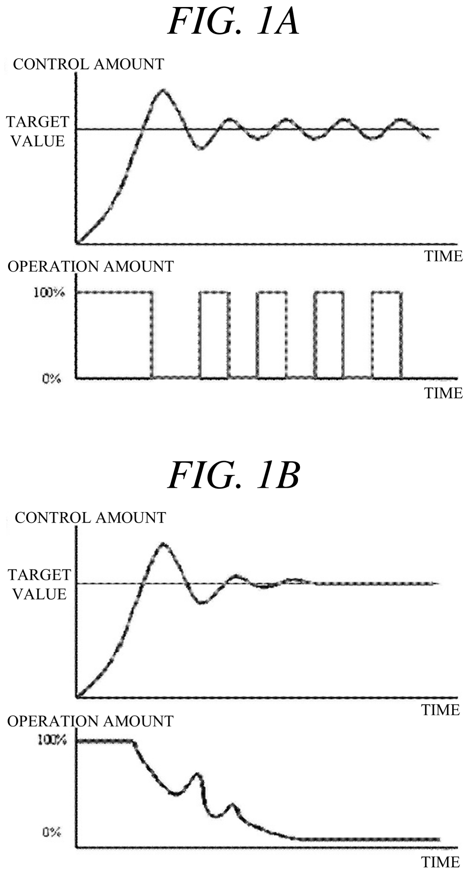

In a denervation according to the prior art, electrical impulses required for nerves, which are a procedure target, are transmitted by PID control (P: Proportional, I: (Proportional) Integral, D: (Proportional) Derivative). Herein, unlike an ON/OFF control method (see **FIG.** 1A), the PID control is a more sophisticated control method using a combination of proportion, integration, and derivation (see **FIG.** 1B).

**FIG. 1A** illustrates an example of the ON/OFF control method, and **FIG. 1B** illustrates an example of the PID control method. Referring to **FIG. 1A**, the ON/OFF control method can be more easily implemented but cannot facilitate convergence to a target. However, referring to **FIG. 1B**, the PID control method can facilitate convergence to a target.

Specifically, the PID control method is a control method that operates proportionally to factors, such as temperature difference, temperature rise and fall, and time, rather than a simple control method that turns on or off at a certain temperature (see **FIG. 1B**). That is, according to the PID control method, whether or not to maintain a voltage is determined by calculating an error between an input reference point and a controlled variable in consideration of variables, and, thus, the PID control method causes fewer errors than the conventional ON/OFF control method.

In the denervation according to the prior art, PID control is performed by using initially set PID control constants. However, in this case, it cannot reflect the characteristics of a tube in a body that vary among individuals.

Therefore, if the PID control constants are set based on the characteristics of a typical medium-sized tube in the body, a temperature of a relatively thin tube in the body may rise rapidly during a surgical procedure, and, thus, it is possible to damage surrounding tissues. On the other hand, a temperature of a relatively thick tube in the body may rise slowly during a surgical procedure, and, thus, the effects of the surgical procedure cannot be properly achieved.

### PRIOR ART DOCUMENT

### PATENT DOCUMENT

Korean Patent No. 10-1615516 (registered on April 20, 2016)

### DISCLOSURE OF THE INVENTION

### PROBLEMS TO BE SOLVED BY THE INVENTION

The present disclosure is conceived to provide an RF energy generator capable of regulating output of RF energy depending on a state of a tube, which is a procedure target, during a surgical procedure, a method of outputting RF energy to an electrode apparatus, and a computer program.

The problems to be solved by the present disclosure are not limited to the above-described problems. There may be other problems to be solved by the present disclosure.

### MEANS FOR SOLVING THE PROBLEMS

According to an aspect of the present disclosure, an RF energy generator that outputs RF energy to an electrode apparatus for blocking or controlling nerves in body includes: a receiving unit configured to receive temperature data of a tube in a body from the electrode apparatus; a controller configured to calculate a temperature value of the tube in the body based on the received temperature data, calculate a temperature variance of the tube in the body based on the calculated temperature value of the tube in the body, and determine an intensity of RF energy through PID control based on the temperature variance of the tube; and an output unit configured to output the RF energy to the electrode apparatus based on the determined intensity of RF energy.

According to another aspect of the present disclosure, a method of outputting RF energy to an electrode apparatus for blocking or controlling nerves in body includes: a process of receiving temperature data of a tube in a body from the electrode apparatus; a process of calculating a temperature value of the tube in the body based on the received temperature data; a process of calculating a temperature variance of the tube in the body based on the calculated temperature value of the tube in the body; a process of determining an intensity of RF energy through PID control based on the temperature variance of the tube; and a process of outputting the RF energy to the electrode apparatus based on the determined intensity of RF energy.

According to yet another aspect of the present disclosure, a computer program stored in a computer-readable storage medium that includes a sequence of instructions to output RF energy to an electrode apparatus for blocking or controlling nerves in body, and the computer program includes a sequence of instructions that, when executed by a computing device, causes the computing device to: receive temperature data of a tube in a body from the electrode apparatus; calculate a temperature value of the tube in the body based on the received temperature data; calculate a temperature variance of the tube in the body based on the calculated temperature value of the tube in the body; determine an intensity of RF energy through PID control based on the temperature variance of the tube; and output the RF energy to the electrode apparatus based on the determined intensity of RF energy.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to the present disclosure, it is possible to control an output intensity of RF energy depending on a state of a tube, which is a procedure target. That is, an intensity of RF energy can be regulated based on a state of a corresponding tube during a surgical procedure. Therefore, it is possible to provide an RF energy generator capable of performing denervation more safely and precisely, a method of outputting RF energy to an electrode apparatus, and a computer program.

### BRIEF DESCRIPTION OF THE DRAWINGS

**FIG. 1A** to **FIG. 1B** are provided to explain a PID control system.
**FIG. 2** is a configuration view of an RF energy generator.
**FIG. 3** shows a process of determining PID control constants.
**FIG. 4** is provided to explain a process of PID control according to the present disclosure.
**FIG. 5** is a flowchart showing a method of outputting RF energy to an electrode apparatus.

### BEST MODE FOR CARRYING OUT THE INVENTION

Hereinafter, embodiments of the present disclosure will be described in detail with reference to the accompanying drawings to be readily implemented by a person with ordinary skill in the art to which the present invention belongs. However, it is to be noted that the present disclosure is not limited to the example embodiments but can be embodied in various other ways. In the drawings, parts irrelevant to the description are omitted in order to clearly explain the present disclosure, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added.

Throughout the whole document, the term "unit" includes a unit implemented by hardware or software and a unit implemented by both of them. One unit may be implemented by two or more pieces of hardware, and two or more units may be implemented by one piece of hardware.

In the present specification, some of operations or functions described as being performed by a device may be performed by a server connected to the device. Likewise, some of operations or functions described as being performed by a server may be performed by a device connected to the server.

Hereafter, an embodiment of the present disclosure will be described in detail with reference to the accompanying drawings.

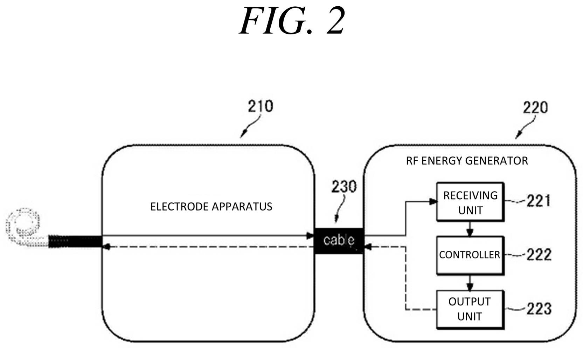

**FIG. 2** is a configuration view of an RF energy generator. Referring to **FIG.** 2, an RF energy generator 220 may include a receiving unit 221, a controller 222, and an output unit 223. However, these components 221 to 223 are illustrated as example components which can be controlled by the RF energy generator 220.

The RF energy generator 220 according to the present disclosure outputs RF energy to an electrode apparatus 210 for blocking or controlling nerves in body. For example, the RF energy generator 220 receives temperature data of a tube in a body, which is a procedure target, from the electrode apparatus 210 through a cable 230. The RF energy generator 220 outputs RF energy to the electrode apparatus 210 based on the received temperature data.

Herein, the RF energy generator 220 can control an output intensity of RF energy based on a temperature variance of the tube in the body. Hereafter, the components of the RF energy generator 220 will be described in detail.

The receiving unit 221 receives the temperature data of the tube in the body from the electrode apparatus 210. For example, the receiving unit 221 receives a temperature signal of the tube in the body measured by a sensor unit *(e.g.,* a thermocouple) of the electrode apparatus 210 through the cable 230. For example, the receiving unit 221 pre-processes the received temperature signal of the tube in the body through conversion of the temperature signal into a temperature value (°C), and transmits the temperature value to the controller 222.

The controller 222 calculates a temperature value of the tube in the body based on the received temperature data. For example, the controller 222 converts the pre-processed signal into a temperature value of the tube in the body.

The controller 222 calculates a temperature variance of the tube in the body based on the calculated temperature value of the tube in the body. For example, the controller 222 may calculate a temperature variance of the tube in the body by comparing temperature data of the tube in the body received from the electrode apparatus 210 at a regular interval. For example, the controller 222 may calculate a temperature variance of the tube by comparing temperature data of the tube obtained before and after output of RF energy to the electrode apparatus 210.

The controller 222 determines an intensity of RF energy through PID control based on the temperature variance of the tube. That is, the controller 222 determines PID constants for PID control based on the temperature variance of the tube. Herein, the PID constants include a proportional constant, an integral constant, and a derivative constant.

Specifically, the controller 222 compares the temperature variance of the tube with a predetermined threshold value. When the temperature variance of the tube is lower than the predetermined threshold value based on the result of the comparison, the controller 222 sets the PID constants as predetermined constants.

The predetermined threshold value is set to include a plurality of values at a regular interval, and the predetermined PID constants are differently set for each interval. For example, the predetermined threshold value is set to include a plurality of values at a regular interval of 0.5°C. For example, the threshold value may be set to 0.5°C, 1.0°C, 1.5°C, or 2.0°C. Herein, when the threshold value is 0.5°C, the PID constants are set to KP1, KI1, and KD1; when the threshold value is 1.0°C, the PID constants are set to KP2, KI2, and KD2; when the threshold value is 1.5°C, the PID constants are set to KP3, KI3, and KD3; and when the threshold value is 2.0°C, the PID constants are set to KP4, KI4, and KD4.

Further, the threshold value may be determined based on an object including the tube, a type of the tube, and a location of the tube. That is, the threshold value may be set according to the characteristics of the procedure target.

The output unit 223 outputs the RF energy to the electrode apparatus 210 based on the determined intensity of RF energy. For example, the output unit 223 outputs the RF energy to the electrode apparatus 210 through the cable 230 at the intensity set according to the PID control constants by the controller 222.

As described above, the RF energy generator 220 according to the present disclosure can regulate the PID control constants depending on the temperature variance of the tube in the body during a surgical procedure. Therefore, the RF energy generator can perform a surgical procedure reflecting the characteristics of a body that vary among individuals. Accordingly, it is possible to perform denervation more safely and precisely.

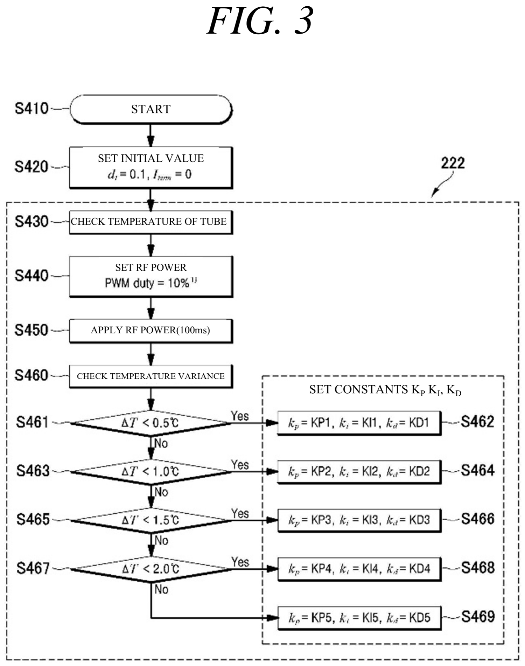

**FIG. 3** is provided to explain a process of determining PID control constants according to the present disclosure. Referring to **FIG.** 3, when a surgical procedure is started (S410), the RF energy generator 220 according to the present disclosure previously sets an initial value except the PID control constants (S420).

The RF energy generator 220 checks a temperature of a tube, which is a procedure target (S430). For example, the RF energy generator 220 checks a temperature of a corresponding tube based on temperature data received from the electrode apparatus 210.

The RF energy generator 220 sets an intensity of RF energy (S440). For example, the RF energy generator 220 may set an intensity of RF energy to about 10%. Herein, 10% is a value that can increase a temperature of a tube in a body by about 0.5°C to about 2°C by a single application of RF energy.

The RF energy generator 220 outputs the RF energy to the electrode apparatus 210 (S450). For example, the RF energy generator 220 outputs the RF energy to the electrode apparatus 210 through a cable at an initially set intensity.

The RF energy generator 220 calculates a temperature variance of the tube after the output of the RF energy (S460). For example, the RF energy generator 220 measures a temperature of the tube RF after a predetermined period of time from the output of the RF energy.

The RF energy generator 220 compares a temperature of the tube before the output of the RF energy with a temperature of the tube after the output of the RF energy (S461). For example, the RF energy generator 220 compares the temperature variance of the tube with a predetermined threshold value, e.g., 0.5°C.

When the temperature variance of the tube, which is a procedure target, is lower than 0.5°C based on the result of the comparison, the RF energy generator 220 sets the PID control constants as predetermined constants (S462). For example, the RF energy generator 220 may set a proportional constant, an integral constant, and a derivative constant of the PID control constants to KP1, KI1, and KD1, respectively.

Meanwhile, when the temperature variance of the tube, which is a procedure target, is equal to or higher than 0.5°C, the RF energy generator 220 compares the temperature variance of the tube with a predetermined threshold value, *e.g.,* 1.0°C (S463).

When the temperature variance of the tube, which is a procedure target, is lower than 1.0°C based on the result of the comparison, the RF energy generator 220 sets the PID control constants as predetermined constants (S464). For example, the RF energy generator 220 may set a proportional constant, an integral constant, and a derivative constant of the PID control constants to KP2, KI2, and KD2, respectively.

Meanwhile, when the temperature variance of the tube, which is a procedure target, is equal to or higher than 1.0°C, the RF energy generator 220 compares the temperature variance of the tube with a predetermined threshold value, e.g., 1.5°C (S465).

When the temperature variance of the tube, which is a procedure target, is lower than 1.5°C based on the result of the comparison, the RF energy generator 220 sets the PID control constants as predetermined constants (S466). For example, the RF energy generator 220 may set a proportional constant, an integral constant, and a derivative constant of the PID control constants to KP3, KI3, and KD3, respectively.

Meanwhile, when the temperature variance of the tube, which is a procedure target, is equal to or higher than 1.5°C, the RF energy generator 220 compares the temperature variance of the tube with a predetermined threshold value, *e.g.,* 2.0°C (S467).

When the temperature variance of the tube, which is a procedure target, is lower than 2.0°C based on the result of the comparison, the RF energy generator 220 sets the PID control constants as predetermined constants (S468). For example, the RF energy generator 220 may set a proportional constant, an integral constant, and a derivative constant of the PID control constants to KP4, KI4, and KD4, respectively.

Meanwhile, when the temperature variance of the tube, which is a procedure target, is equal to or higher than 2.0°C, the RF energy generator 220 sets the PID control constants as predetermined constants (S469). For example, the RF energy generator 220 may set a proportional constant, an integral constant, and a derivative constant of the PID control constants to KP5, KI5, and KD5, respectively.

As described above, the RF energy generator 220 according to the present disclosure can set the PID control constants based on the temperature variance of nerves of the tube, which is a procedure target during a surgical procedure and control the intensity of RF energy. Therefore, the RF energy generator 220 can perform a surgical procedure more safely and precisely in consideration of a state (temperature) of a tube that varies among individuals.

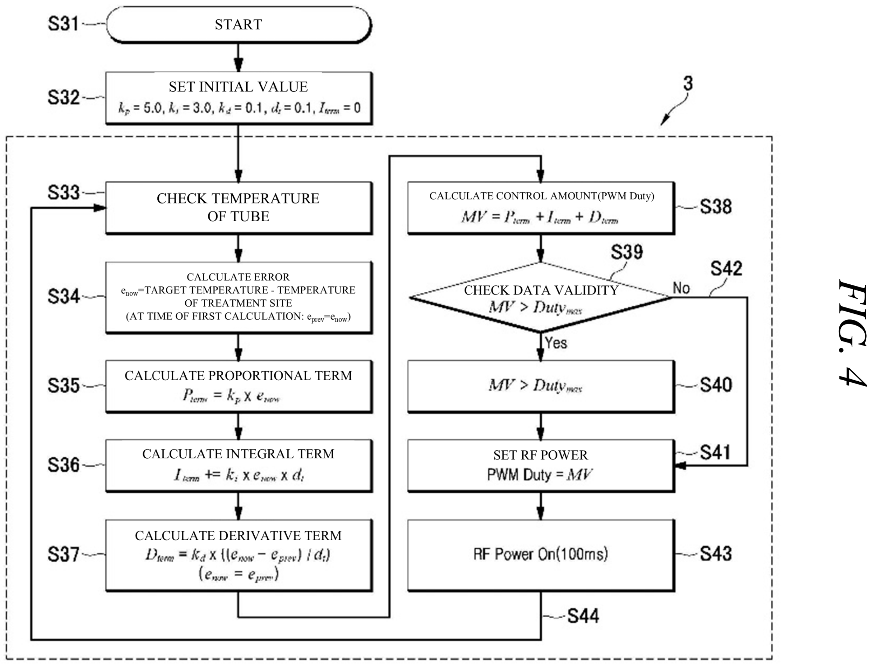

**FIG. 4** shows a process of PID control according to the present disclosure. Referring to **FIG.** 4, when a surgical procedure is started (S31), the RF energy generator 220 sets an initial value (S32). For example, the RF energy generator 220 may set a proportional constant, an integral constant, and a derivative constant as PID control constants determined during the process of **FIG.** 3 to 5.0, 3.0, and 0.1, respectively.

The RF energy generator 220 checks a temperature of a tube, which is a procedure target (S33). For example, the RF energy generator 220 checks a temperature of a corresponding tube based on temperature data received from the electrode apparatus 210.

The RF energy generator 220 calculates an error between a predetermined target temperature and the temperature of the tube (S34). For example, the RF energy generator 220 calculates an error between a predetermined target temperature and the temperature of the tube received from the electrode apparatus 210 to achieve the effects of the surgical procedure.

The RF energy generator 220 calculates a proportional term from the PID control constants based on the calculated error (S35), calculates an integral term from the PID control constants based on the calculated error (S36), and calculates a derivative term from the PID control constants based on the calculated error (S37).

The RF energy generator 220 calculates a PID control amount from the calculated proportional term, integral term and derivative term (S38). The RF energy generator 220 checks data validity by comparing the calculated PID control amount with a predetermined threshold value (S39).

When the calculated PID control amount is higher than the threshold value, the RF energy generator 220 sets the predetermined threshold value to the PID control amount (S40) and sets an intensity of RF energy based on the threshold value (S41). Meanwhile, when the PID control amount is lower than the threshold value, the RF energy generator 220 determines the intensity of the RF energy based on the calculated PID control amount (S42).

Further, the RF energy generator 220 outputs the RF energy to the electrode apparatus. The RF energy generator 220 continues to check a temperature of the tube, which is a procedure target, while outputting the RF energy to the electrode apparatus (S44).

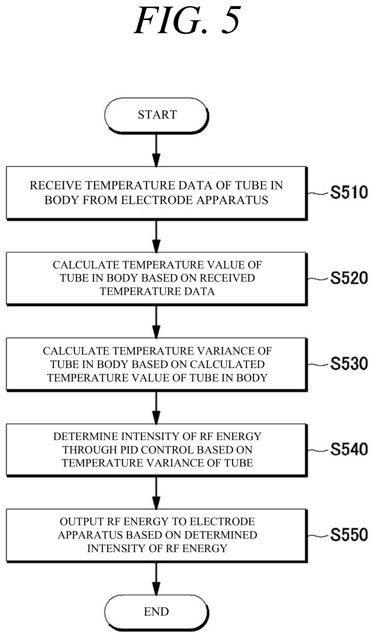

**FIG. 5** is a flowchart showing a method of outputting RF energy to an electrode apparatus. The RF energy output method illustrated in **FIG.** 5 includes the processes time-sequentially performed according to the embodiment illustrated in **FIG. 2** to **FIG. 4****.** Therefore, the descriptions of the processes may also be applied to the method of outputting RF energy from an RF energy generator to an electrode apparatus according to the embodiment illustrated in **FIG. 2** to **FIG. 4** even though they are omitted hereinafter.

In an operation S510, the RF energy generator 220 may receive temperature data of a tube in a body from the electrode apparatus.

In an operation S520, the RF energy generator 220 may calculate a temperature value of the tube in the body based on the received temperature data.

In an operation S530, the RF energy generator 220 may calculate a temperature variance of the tube in the body based on the calculated temperature value of the tube in the body.

In an operation S540, the RF energy generator 220 may determine an intensity of RF energy through PID control based on the temperature variance of the tube.

In an operation S550, the RF energy generator 220 may output the RF energy to the electrode apparatus based on the determined intensity of RF energy.

In the descriptions above, the operations S510 to S550 may be divided into additional operations or combined into fewer operations depending on an embodiment. In addition, some of the operations may be omitted and the sequence of the operations may be changed if necessary.

The method of outputting RF energy from an RF energy generator to an electrode apparatus described above with reference to **FIG.** 2 to **FIG.** 5 can be implemented as a computer program stored in a computer-readable storage medium to be executed by a computer or a storage medium including instructions executable by a computer. Further, the method of outputting RF energy from an RF energy generator to an electrode apparatus described above with reference to **FIG.** 2 to **FIG.** 5 can be implemented as a computer program stored in a computer-readable storage medium to be executed by a computer.

A computer-readable storage medium can be any usable medium which can be accessed by the computer and includes all volatile/non-volatile and removable/non-removable media. Further, the computer-readable storage medium may include all computer storage and communication media. The computer storage media include all volatile/non-volatile and removable/non-removable media embodied by a certain method or technology for storing information such as computer-readable instruction code, a data structure, a program module or other data.

The above description of the present disclosure is provided for the purpose of illustration, and it would be understood by a person with ordinary skill in the art that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment, and it should be understood that all modifications and embodiments conceived from the meaning and scope of the claims and their equivalents are included in the scope of the present disclosure.

## Claims

1. An RF energy generator that outputs RF energy to an electrode apparatus for blocking or controlling nerves in body, comprising:
a receiving unit configured to receive temperature data of a tube in a body from the electrode apparatus;
a controller configured to calculate a temperature value of the tube in the body based on the received temperature data, calculate a temperature variance of the tube in the body based on the calculated temperature value of the tube in the body, and determine an intensity of RF energy through PID control based on the temperature variance of the tube; and
an output unit configured to output the RF energy to the electrode apparatus based on the determined intensity of RF energy.

2. The RF energy generator of Claim 1,
wherein the controller determines PID constants for PID control based on the temperature variance of the tube.

3. The RF energy generator of Claim 2,
wherein the controller compares the temperature variance of the tube with a predetermined threshold value, and
when the temperature variance of the tube is lower than the predetermined threshold value based on the result of the comparison, the controller sets the PID constants as predetermined constants.

4. The RF energy generator of Claim 3,
wherein the predetermined threshold value is set to include a plurality of values at a regular interval, and
the predetermined PID constants are differently set for each interval.

5. The RF energy generator of Claim 3,
wherein the PID constants include a proportional constant, an integral constant, and a derivative constant.

6. The RF energy generator of Claim 3,
wherein the threshold value is determined based on an object including the tube, a type of the tube, and a location of the tube.

7. A method of outputting RF energy to an electrode apparatus for blocking or controlling nerves in body, comprising:
a process of receiving temperature data of a tube in a body from the electrode apparatus;
a process of calculating a temperature value of the tube in the body based on the received temperature data;
a process of calculating a temperature variance of the tube in the body based on the calculated temperature value of the tube in the body;
a process of determining an intensity of RF energy through PID control based on the temperature variance of the tube; and
a process of outputting the RF energy to the electrode apparatus based on the determined intensity of RF energy.

8. The method of outputting RF energy of Claim 7,
wherein the process of determining an intensity of RF energy includes:
a process of determining PID constants for PID control based on the temperature variance of the tube.

9. The method of outputting RF energy of Claim 8,
wherein the process of determining an intensity of RF energy further includes:
a process of comparing the temperature variance of the tube with a predetermined threshold value; and
a process of setting the PID constants as predetermined constants when the temperature variance of the tube is lower than the predetermined threshold value based on the result of the comparison.

10. The method of outputting RF energy of Claim 9,
wherein the predetermined threshold value is set to include a plurality of values at a regular interval, and
the predetermined PID constants are differently set for each interval.

11. The method of outputting RF energy of Claim 9,
wherein the PID constants include a proportional constant, an integral constant, and a derivative constant.

12. The method of outputting RF energy of Claim 9,
wherein the threshold value is determined based on an object including the tube, a type of the tube, and a location of the tube.

13. A computer program stored in a computer-readable storage medium that includes a sequence of instructions to output RF energy to an electrode apparatus for blocking or controlling nerves in body,
wherein the computer program includes a sequence of instructions that, when executed by a computing device, causes the computing device to:
receive temperature data of a tube in a body from the electrode apparatus;
calculate a temperature value of the tube in the body based on the received temperature data;
calculate a temperature variance of the tube in the body based on the calculated temperature value of the tube in the body;
determine an intensity of RF energy through PID control based on the temperature variance of the tube; and
output the RF energy to the electrode apparatus based on the determined intensity of RF energy.
